(19) Europäisches Patentamt
European Patent Office
Office européen des brevets

(11) Veröffentlichungsnummer: **0 508 261 A2**

(12) **EUROPÄISCHE PATENTANMELDUNG**

(21) Anmeldenummer: **92105448.2**

(51) Int. Cl.5: **C07C 53/50**, C07C 51/62

(22) Anmeldetag: **30.03.92**

(30) Priorität: **12.04.91 DE 4111908**

(43) Veröffentlichungstag der Anmeldung:
**14.10.92 Patentblatt 92/42**

(84) Benannte Vertragsstaaten:
**BE CH DE FR GB IT LI NL**

(71) Anmelder: **BAYER AG**
**W-5090 Leverkusen 1 Bayerwerk(DE)**

(72) Erfinder: **Schubart, Rüdiger, Dr.**
**An der Engelsfuhr 27**
**W-5060 Bergisch Gladbach(DE)**

(54) **Verfahren zur Herstellung von beta-Halogen-tert.-alkylcarbonsäurechloriden.**

(57) Die Erfindung betrifft ein neues Verfahren zur Herstellung von bekannten β-Halogen-tert.-alkylcarbonsäurechloriden der allgemeinen Formel (I)

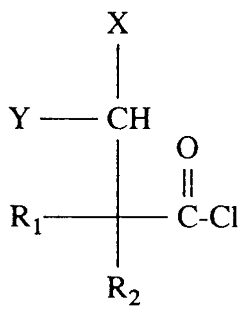

(I),

in welcher
X für Chlor steht,
Y für Wasserstoff oder Chlor steht,
$R_1$ für jeweils geradkettiges oder verzweigtes niederes Alkyl oder Halogenalkyl steht und
$R_2$ für jeweils geradkettiges oder verzweigtes niederes Alkyl oder Halogenalkyl, oder für gegebenenfalls durch Halogen und/oder Trifluormethyl substituiertes Phenyl steht,
wobei man tert.-Alkylcarbonsäurechloride der allgemeinen Formel (II)

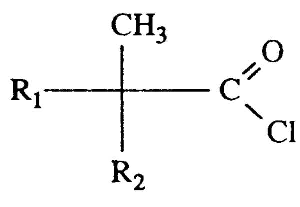

(II),

in welcher
$R^1$ und $R^2$ die oben angegebene Bedeutung haben, mit elementarem Chlor, gegebenenfalls unter Belichtung oder in Gegenwart von Radikalkatalysatoren, umsetzt.

EP 0 508 261 A2

Die Erfindung betrifft ein neues Verfahren zur Herstellung von bekannten β-Halogen-tert.-alkylcarbonsäurechloriden, die als Zwischenprodukte einerseits für die Synthese von Kautschukhilfsmitteln, andererseits auch als wichtige Zwischenprodukte zur Synthese herbizider Wirkstoffe verwendet werden können (vgl. US P 4 033 904.1).

Es ist bereits bekannt, daß man z.B. β-Monochlor-tert.-butylcarbonsäurechlorid durch Umsetzen von tert.-Butylcarbonsäurechlorid mit Sulfurylchlorid (J. Amer. Chem. Soc. 62, 925/1940) in Gegenwart eines Chlorierungskatalysators erhält

Üblicherweise verwendet man dabei Peroxide oder Azoverbindungen als Katalysatoren, oder man führt die Reaktion unter Bestrahlung von UV-Licht mit Geräten aus, die allgemein für UV-Licht katalysierte Reaktionen verwendet werden.

Nachteilig an dieser Synthese sind die schlechten Ausbeuten an gewünschtem Monochlor-tert.-butylcarbonsäurechlorid. Ferner ist auch die Gasphasenchlorierung des tert.-Butylcarbonsäurechlorids bekannt [DE-OS 3 618 928 A1 (1986)].

Dieses Verfahren hat jedoch den erheblichen Nachteil, daß die Chlorierung nur extrem langsam durchgeführt werden kann, da in jedem Fall ein Chlorüberschuß vermieden werden muß. Dieser führt nämlich zur bevorzugten Bildung von Nebenprodukten, so daß nach dieser Herstellungsvariante keine großen Produktmengen pro Zeiteinheit synthetisiert werden können. Der wichtige Faktor der Raum/Zeit-Ausbeute liegt hierbei in einem sehr ungünstigen Verhältnis vor.

Mit der vorliegenden Erfindung wird ein Verfahren vorgestellt, das es gestattet, β-Halogen-tert.-alkylcarbonsäurechloride der Formel (I)

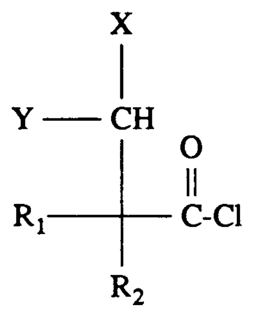

(I),

in welcher

- X für Chlor steht,
- Y für Wasserstoff oder Chlor steht,
- $R_1$ für jeweils geradkettiges oder verzweigtes niederes Alkyl oder Halogenalkyl steht und
- $R_2$ für jeweils geradkettiges oder verzweigtes niederes Alkyl oder Halogenalkyl, oder für gegebenenfalls durch Halogen und/oder Trifluormethyl substituiertes Phenyl steht,

mit hoher Selektivität und Ausbeute bei gleichzeitig optimaler Raum/Zeit-Ausbeute zu synthetisieren, indem man
tert.-Alkylcarbonsäurechloride der Formel (II)

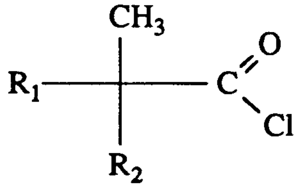

(II),

in welcher
$R^1$ und $R^2$ die oben angegebene Bedeutung haben, mit elementarem Chlor, gegebenenfalls unter Belichtung oder in Gegenwart von Radikalkatalysatoren, in einer geeigneten Apparatur (siehe hierzu beispielsweise DE 2 716 896) umsetzt.

Es ist ausgesprochen überraschend, daß die Reaktion in hohen Ausbeuten bei hoher Selektivität und gleichzeitig optimaler Raum/Zeit-Ausbeute abläuft Aus dem oben aufgeführten Stand der Technik mußte vielmehr mit einer Reihe von Nebenprodukten bei gleichzeitig erheblich geringerer Produktmenge pro

Zeiteinheit, d.h. mit einer sehr viel ungünstigeren Raum/Zeit-Ausbeute gerechnet werden.

Mit Hilfe des erfindungsgemäßen Verfahrens erhält man bevorzugt Verbindungen der Formel (I), in welcher $R_1$ und $R_2$ niederes Alkyl wie Methyl oder Ethyl darstellen.

Ganz besonders bevorzugt lassen sich nach dem erfindungsgemäßen Verfahren die Verbindungen der Formel (I) synthetisieren, in denen X für Chlor und Y für Wasserstoff steht, d.h. $\beta$-monochlorierte tert.-Alkylcarbonsäurechloride.

Das erfindungsgemäße Verfahren läßt sich bei Verwendung von tert.-Butylcarbonsäurechlorid durch folgendes Formelschema darstellen:

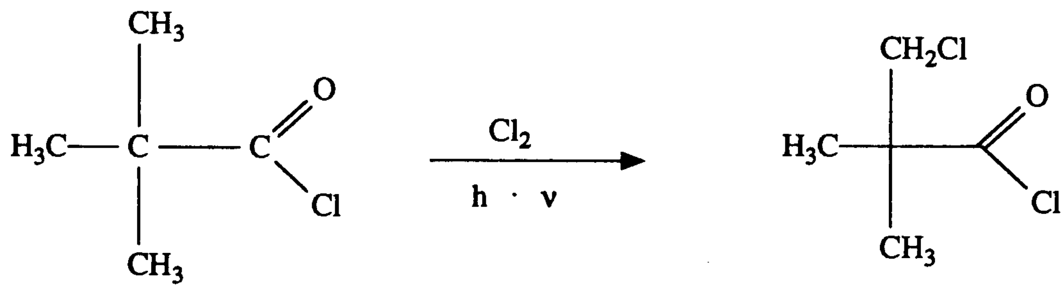


Das erfindungsgemäße Verfahren wird bevorzugt in Substanz, also ohne Verdünnungsmittel, durchgeführt. Es kann aber auch in Gegenwart von geeigneten inerten Lösungsmitteln wie Chloroform oder Tetrachlormethan durchgeführt werden.

Die direkte Chlorierung der Verbindungen der Formel (II) kann gegebenenfalls durch Radikalkatalysatoren wie Peroxide oder Azoverbindungen in üblicher Weise durch ständige Zugabe unterstützt werden. Chemisch sauberer ist jedoch die Verwendung von UV-Licht zur Chlorierungskatalyse, wobei übliche UV-Quellen eingesetzt werden können.

Die Bestrahlung kann beispielsweise mit einem wassergekühlten Quecksilber-Hochdruckbrenner durchgeführt werden, wobei die Halogenierungslampe sowohl als Tauchlampe eingesetzt, als auch von außen angebracht werden kann. Bei der Anbringung der Lampe ist darauf zu achten, daß möglichst viel Licht in die Halogenierungszone gelangt. Alle für solche Halogenierungen üblichen Quecksilber-Hochdruckbrenner lassen sich für das erfindungsgemäße Verfahren einsetzen. Natürlich sind auch andere, für solche Halogenierungen geeignete Lampen, einsetzbar.

Als Katalysatoren verwendet man vorzugsweise Peroxide, wie beispielsweise Cumylperoxid oder Benzoylperoxid, oder Azoverbindungen wie beispielsweise Azoisobuttersäurenitril (AIBN).

Die Reaktionstemperaturen können bei der Durchführung des erfindungsgemäßen Verfahrens in einem größeren Bereich variiert werden. Im allgemeinen arbeitet man bei Temperaturen zwischen 40 ° C und 120 ° C, bevorzugt zwischen 60 ° C und 110 ° C, besonders bevorzugt zwischen 85 ° C und 100 ° C. Insbesondere arbeitet man bei Temperaturen, die im Bereich der Siedetemperatur des zu chlorierenden tert.-Alkylcarbonsäurechlorides bzw. des Verdünnungsmittels liegen.

Das erfindungsgemäße Verfahren kann bei Normaldruck oder bei Überdruck bis zu 2000 mbar durchgeführt werden. Im allgemeinen arbeitet man bei Normaldruck.

In einer entsprechend geeigneten Apparatur, die beispielsweise aus einem Destillationskolben, einer Füllkörperkolonne, einem Kühler und der Chlorierungseinrichtung besteht, wird zunächst Edukt verdampft Dieses gelangt durch die Kolonne zum Kühler und kondensiert dort. Das kondensierte Edukt kann nun ganz oder teilweise durch die Chlorierungszone, die gleichzeitig belichtet und mit Chlor beschickt wird, geführt werden. Das in dieser Chlorierungszone entstandene Reaktionsgemisch, bestehend aus Edukt und Produkt der Formel (I) wird nun der Trennkolonne zwecks Trennung in die Komponenten zugeführt.

Eine entsprechend geeignete Apparatur ist zum Beispiel in der DE 2 716 896 beschrieben. Prinzipiell sind aber auch andere Apparaturen für diesen Kreislaufprozeß einsetzbar.

Das erfindungsgemäße Verfahren kann in zwei unterschiedlichen Varianten durchgeführt werden und zwar als sogenannte Batchchlorierung (diskontinuierliche Chlorierung) (Variante 1) oder in Form einer kontinuierlichen Halogenierung (Variante 2).

Bei Variante 1 des erfindungsgemäßen Verfahrens führt man ständig gleichzeitig etwa äquimolare Mengen an Edukt (z.B. tert.-Butylcarbonsäurechlorid) und Chlor getrennt einer gefluteten Reaktionszone unter Belichtung oder unter dauernder Zugabe eines üblichen Katalysators zu, wobei das entstehende Reaktionsgemisch, welches Edukt der Formel (II) und Produkt der Formel (I) enthält, der Reaktionszone gleichzeitig entnommen wird.

Dieses Gemisch wird einer Kolonne zur Fraktionierung zugeführt und nicht halogeniertes Edukt der Formel (II) sofort abgetrennt und erneut der Halogenierungszone zugeführt. Dies wird solange wiederholt, bis nahezu das gesamte Edukt umgesetzt ist.

Zur Durchführung von Variante 2 des Verfahrens (kontinuierliche Halogenierung) setzt man für den Fall, daß die monohalogenierten Verbindungen der Formel (I) erhalten werden sollen, pro Mol tert.-Alkylcarbonsäurechlorid der Formel (II) insgesamt im allgemeinen äquivalente Mengen bzw. einen geringfügigen Überschuß an Halogen ein. Für den Fall, daß eine dihalogenierte Verbindung der Formel (I) hergestellt werden soll, setzt man pro Mol tert.-Alkylcarbonsäurechlorid im allgemeinen äquivalente Mengen oder einen Überschuß, bevorzugt bis 2,25 Mol bzw. besonders bevorzugt bis 2,05 Mol an Halogen ein. Vorzugsweise setzt man bei der kontinuierlichen Halogenierung zur Herstellung der dihalogenierten Verbindungen einen geringen Überschuß an Halogen ein.

Falls das Verfahren in Gegenwart von Katalysatoren durchgeführt wird, setzt man pro Mol tert.-Alkylcarbonsäurechlorid der Formel (II) im allgemeinen 0,001 bis 1,5 %, bevorzugt 0,02 bis 1 % und besonders bevorzugt 0,1 bis 0,5 %, an Katalysator ständig zu.

Im Falle einer kontinuierlichen Fahrweise (Variante 2) wird dem abgetrennten Edukt aus einem Vorratsgefäß frisches Edukt hinzugefügt und erneut in die Halogenierungszone geleitet, so daß die Halogenierung beliebig lange fortgeführt werden kann.

Man kann die Halogenierungsreaktion des gesamten eingesetzten tert.-Alkylcarbonsäurechlorids bis zu fast vollständigem Umsatz durchführen, in der Regel wird bei der Batchfahrweise (Variante 1) bis zu ≧ 95 % Umsatz chloriert. Dann unterbricht man die Halogenierungsreaktion. Natürlich könnte auch bereits zu einem früheren Umsatzzeitpunkt, z.B. bei etwa 60 % Umsatz, abgebrochen werden. Umsatz wird hier definiert als 100 % Edukt minus noch vorhandenes Edukt. Das Reaktionsgemisch wird dann fraktioniert, um nicht halogeniertes Edukt zurückzugewinnen, welches erneut der Reaktion zugeführt wird.

Das halogenierte tert.-Alkylcarbonsäurechlorid, d.h. Verbindungen der Formel (I), wird am Boden der Apparatur abgesondert und im Falle genügender Reinheit direkt für weitere Umsetzungen verwendet, oder ständig der Apparatur entnommen und einer Feinfraktionierung unterworfen.

Auf diese Weise entsteht ein kontinuierlich arbeitender Prozeß, bei dem ständig Reaktionsgemisch entnommen und frisches Edukt sowie Chlor dem Kreisprozeß zugeführt werden.

Die Vermischung des Halogens in der gefluteten Reaktionszone kann z.B. durch Rühren, durch die Strömung der Reaktionspartner bei der Zugabe in einem Venturi-Düsenrohr oder eine Kombination dieser Möglichkeiten, sowie anderen möglichen geeigneten Vorrichtungen erfolgen.

Auf diese Weise erhält man gezielt mono- bzw. dihalogenierte Verbindungen der Formel (I) in hoher Ausbeute und Reinheit und hält die Bildung von Nebenprodukten gering. Mit einer relativ Kleinen Apparatur lassen sich durch die kontinuierliche Verfahrensweise pro Zeiteinheit somit große Mengen an gewünschtem Produkt herstellen.

Bei der batchweisen Variante 1 und auch bei der kontinuierlichen Reaktionsführung (Variante 2) wird die Chlorierung des durch die Chlorierungszone laufenden tert.-Alkylcarbonsäurechlorids im allgemeinen bis zu einem Umsatz zwischen 0,1 und 30 %, vorzugsweise zwischen 2 und 20 %, besonders bevorzugt zwischen 5 und 15 %, durchgeführt, so daß insgesamt Chlor und tert.-Alkylcarbonsäurechlorid der Apparatur zugeführt und chloriertes tert.-Alkylcarbonsäurechlorid praktisch ohne Edukt der Apparatur entnommen werden. Die Reaktion kann sowohl bei der Batchhalogenierung, als auch bei der kontinuierlichen Halogenierung unterbrochen und der Prozeß nach einiger Zeit fortgesetzt werden. Das Reaktionsgemisch kann auch zwischengelagert und erst nach einiger Zeit weiterverarbeitet werden.

Prinzipiell sind beide Verfahrensvarianten auch zur Bromierung von tert.-Alkylcarbonsäurechloriden anwendbar.

Der Vorteil dieser Verfahrensweise liegt darin, daß der Anteil der unbrauchbaren Nebenprodukte und damit die Menge des Materialverlustes (d.h. Verlust an Edukt der Formel II) deutlich geringer wird.

Nach dieser Herstellungsweise läßt sich die Ausbeute an $\beta$-Halogen-tert.-alkylcarbonsäurechlorid aus einer bestimmten Menge tert.-Alkylcarbonsäurechlorid pro Raum- und Zeiteinheit deutlich verbessern.

Das erfindungsgemäße Verfahren wird durch folgendes Beispiel veranschaulicht.

In einer Labor-Apparatur, die für eine batchweise oder für eine kontinuierliche Chlorierung geeignet ist, werden 689 g (5,7 mol) tert.-Butylcarbonsäurechlorid vorgelegt und wie oben beschrieben durch Teilchlorierung eines im Kreis geführten Eduktstromes in einer völlig gefluteten Reaktionszone langsam unter UV-Belichtung ($H_2$-Hochdruckbrenner) chloriert, wobei für die Chlorierungstemperatur die Siedetemperatur des tert.-Butylcarbonsäurechlorids eingehalten, oder nur wenig unterschritten wird. Wenn der Umsatz ungefähr ≧ 95 % erreicht hat, kann die Reaktion abgebrochen werden (Variante 1), oder es kann tert.-Butylcarbonsäurechlorid und Chlor im Verhältnis 1:1,06 ständig der Apparatur bzw. der Chlorierungszone zugeführt und chloriertes Produkt nahezu völlig ohne Edukt der Apparatur entnommen werden (Variante 2).

Aus 689 g (- 5,7 Mol) tert.-Butylcarbonsäurechlorid erhält man bei einem Umsatz von - 98 % ein Rohprodukt, das folgende Zusammensetzung aufweist (GC, in Flächenprozent):

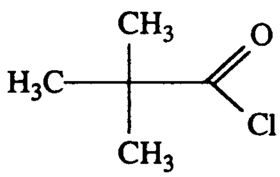

2 %

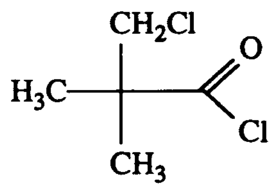

90 %

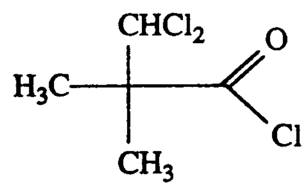

3,2 %

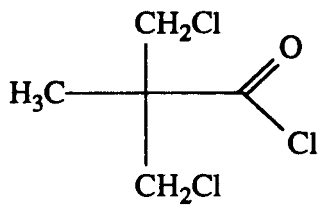

3,5 %

Nach Fraktionierung des Rohprodukts erhält man 780 g Monochlor-tert.-butylcarbonsäurechlorid mit einem Siedepunkt von 60 ° C bei 20 mbar und einer Reinheit von 98 %. Das entspricht einer Ausbeute von - 90 % der Theorie. 2 % Edukt können wieder zurückgewonnen werden und werden folglich von der Berechnung der Ausbeute abgezogen.

**Patentansprüche**

**1.** Verfahren zur Herstellung von β-Halogen-tert.-alkylcarbonsäurechloriden der Formel I

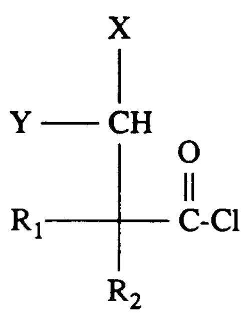

(I),

in welcher

X für Chlor steht,

Y für Wasserstoff oder Chlor steht,

$R_1$ für jeweils geradkettiges oder verzweigtes niederes Alkyl oder Halogenalkyl steht und

$R_2$ für jeweils geradkettiges oder verzweigtes niederes Alkyl oder Halogenalkyl, oder für gegebenenfalls durch Halogen und/oder Trifluormethyl substituiertes Phenyl steht,

dadurch gekennzeichnet, daß man tert.-Alkylcarbonsäurechloride der Formel (II)

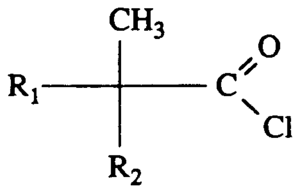

(II),

in welcher

$R^1$ und $R^2$ die oben angegebene Bedeutung haben, mit elementarem Chlor, gegebenenfalls unter Belichtung oder in Gegenwart von Radikalkatalysatoren in einer geeignet Apparatur umsetzt.

2. Verfahren gemäß Anspruch 1, dadurch gekennzeichnet, daß $R^1$ für Methyl, Ethyl, n- oder iso-Propyl, n-, iso-, sec-, tert.-Butyl und deren monohalogenierte Derivate steht und $R^2$ für Methyl, Ethyl, n- oder iso-Propyl, n-, iso-, sec.- oder tert.-Butyl und deren monohalogenierte Derivate oder für gegebenenfalls einfach bis dreifach, gleich oder verschieden durch Fluor, Chlor, Brom und/oder Trifluormethyl substituiertes Phenyl steht.

3. Verfahren gemäß Anspruch 1, dadurch gekennzeichnet daß $R^1$ für Methyl oder Ethyl und $R^2$ für Methyl, Ethyl oder Phenyl stehen.

4. Verfahren gemäß Anspruch 1, dadurch gekennzeichnet, daß man Peroxide oder Azoverbindungen als Katalysatoren verwendet.

5. Verfahren gemäß Anspruch 1, dadurch gekennzeichnet, daß man die Umsetzung als Batchchlorierung durchführt (Variante 1).

6. Verfahren gemäß Anspruch 1, dadurch gekennzeichnet, daß man die Reaktion als kontinuierliche Halogenierung durchführt (Variante 2).

7. Verfahren gemäß Anspruch 1, dadurch gekennzeichnet, daß man die Chlorierung bis zu einem Umsatz zwischen 0,1 und 30 % des durch die Chlorierungszone laufenden tert.-Alkylcarbonsäurechlorids durchführt.